(19) **Europäisches Patentamt / European Patent Office / Office européen des brevets**

(11) **EP 4 588 497 B1**

(12) **EUROPEAN PATENT SPECIFICATION**

(45) Date of publication and mention of the grant of the patent:
**17.12.2025 Bulletin 2025/51**

(21) Application number: **24754211.1**

(22) Date of filing: **05.06.2024**

(51) International Patent Classification (IPC):
**A61M 16/00** *(2006.01)*

(52) Cooperative Patent Classification (CPC):
(C-Sets available)
**A61M 16/0009; A61M 16/0096; A61M 16/024;**
A61M 16/12; A61M 16/204; A61M 16/205;
A61M 2016/0027; A61M 2016/0036;
A61M 2016/0039; A61M 2016/0042;
A61M 2202/0208; A61M 2202/0225;
A61M 2205/3334; A61M 2205/3344;
A61M 2230/205                     (Cont.)

(86) International application number:
**PCT/CN2024/097388**

(87) International publication number:
**WO 2025/112415 (05.06.2025 Gazette 2025/23)**

(54) **HIGH-FREQUENCY BREATHING CONTROL SYSTEM FOR HARMONIC CONTROL OF POSITIVE AND NEGATIVE PRESSURE VENTILATION, AND BREATHING MACHINE**

HOCHFREQUENZ-ATEMSTEUERUNGSSYSTEM UND BEATMUNGSGERÄT MIT OBERWELLENGESTEUERTER POSITIV- UND UNTERDRUCKBEATMUNG

SYSTÈME DE COMMANDE RESPIRATOIRE HAUTE FRÉQUENCE À DES FINS DE COMMANDE HARMONIQUE DE VENTILATION À PRESSION POSITIVE ET NÉGATIVE, ET MACHINE RESPIRATOIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **30.11.2023 CN 202311615667**

(43) Date of publication of application:
**23.07.2025 Bulletin 2025/30**

(73) Proprietor: **Guangzhou Landswick Medical Technologies Ltd.**
**Guangzhou, Guangdong 510000 (CN)**

(72) Inventors:
• **DONG, Hui**
  **Guangzhou, Guangdong 510000 (CN)**
• **SUN, Caixin**
  **Guangzhou, Guangdong 510000 (CN)**
• **ZHAO, Longchao**
  **Guangzhou, Guangdong 510000 (CN)**

(74) Representative: **Santarelli**
**Tour Trinity**
**1 bis Place de la Défense**
**92400 Courbevoie (FR)**

(56) References cited:
WO-A1-2021/179215          CN-A- 104 640 590
CN-A- 110 464 951           CN-A- 114 949 520
CN-A- 116 764 051           CN-A- 117 504 076
DE-A1- 102016 122 187    DE-A1- 102016 122 187
US-A1- 2021 016 029

(52) Cooperative Patent Classification (CPC): (Cont.)

C-Sets
A61M 2202/0208, A61M 2202/0007;
A61M 2202/0225, A61M 2202/0007;
A61M 2230/205, A61M 2230/005

## Description

### Technical Field

This invention relates to the field of ventilator technology, more specifically, this invention relates to a high-frequency respiratory control system and ventilator with harmonic-controlled positive and negative pressure ventilation.

### Background Technology

High-frequency ventilation (HFV) uses a frequency significantly higher than the physiological respiratory rate, with extremely low tidal volumes for ventilation.

High-frequency ventilation is a ventilation technique that uses a ventilation frequency more than 4 times the normal respiratory rate, with tidal volumes close to or lower than the anatomical dead space volume. According to the differences in ventilation principles, high-frequency ventilation can be divided into three different types, namely high-frequency positive pressure ventilation, high-frequency jet ventilation, and high-frequency oscillatory ventilation.

High-frequency positive pressure ventilation (HFPPV) often uses the gas valve method, which controls gas flow with a high-frequency pneumatic valve, periodically delivering gas into the inspiratory tube; the ventilation frequency typically chosen is 60-120 times/min (1-2Hz), with a tidal volume of 3-5ml/kg, and an inspiratory to expiratory time ratio of less than 0.3.

High-frequency jet ventilation (HFJV) uses a high-pressure gas source to deliver gas into the airway through a thin catheter in a jet manner; the typically chosen gas source pressure is 103.4-344.7kPa, with a ventilation frequency of 120-300 times/min (2-5Hz), and a tidal volume of 2-5ml/kg.

High-frequency oscillatory ventilation (HFO) promotes gas movement in and out of the airway through the reciprocating motion of a piston pump or vibration waves from a speaker; the vibration frequency can reach 300-3600 times/min (5-60Hz), with a tidal volume of 1-3ml/kg.

WO 2021/179215 A1 discloses a high-frequency gas flow generator of the prior-art.

Although the tidal volume of high-frequency ventilation is low, its ventilation frequency is high, which can achieve a higher minute ventilation volume to reach the treatment goal.

Currently, traditional high-frequency gas flow generators are large in volume, complex in structure, and carbon dioxide tends to accumulate at the gas supply end, which is not conducive to elimination, and the tidal volume control accuracy is low.

Therefore, it is necessary to propose a high-frequency respiratory control system, method, and ventilator with harmonic-controlled positive and negative pressure ventilation to at least partially solve the problems existing in the current technology.

### Summary of the Invention

A series of simplified concepts are introduced in the summary of the invention section, which will be further detailed in the specific implementation section. The summary of the invention section is not intended to limit the key features and necessary technical features of the technical solution to be protected, nor is it intended to determine the scope of protection of the technical solution to be protected. The scope of protection is defined by the appended claims.

To at least partially solve the above problems, this invention provides a high-frequency respiratory control system with harmonic-controlled positive and negative pressure ventilation, including: an inspiratory tube section and an expiratory tube section, where the inlet A of the inspiratory tube section is connected to a gas source, the outlet B of the expiratory tube section is connected to a vacuum source, and the outlet C of the inspiratory tube section and the inlet D of the expiratory tube section are both connected to the gas supply end through a third flowmeter and a pressure meter; a control module, used to control the alternate opening and closing of the inspiratory and expiratory tube sections, providing high-frequency gas supply to the patient.

The inspiratory tube section includes: a first safety on-off valve, a first proportional valve, a first high-frequency on-off valve, and a first flowmeter connected in sequence, where the first safety on-off valve is connected to the gas source.

The expiratory tube section includes: a second safety on-off valve, a second flowmeter, a second proportional valve, and a second high-frequency on-off valve connected in sequence, where the second safety on-off valve is connected to the vacuum source.

Preferably, the gas source is one of a pure oxygen source, a mixed gas source of air and oxygen, or a mixed gas source of carbon dioxide and oxygen.

The control module includes:

a setting unit, used to preset the required positive pressure flow and positive pressure output time of the inspiratory tube section, negative pressure flow and negative pressure input time of the expiratory tube section, as well as the safety flow standard and safety pressure standard of the gas supply end;

an acquisition unit, used to obtain in real-time the first flow value from the first flowmeter, the second flow value from the second flowmeter, the third flow value from the third flowmeter, and the pressure value from the pressure meter;

an adjustment unit, used to adjust the opening of the first proportional valve based on the first flow value obtained by the acquisition unit, to make the first flow value meet the preset positive pressure flow; adjust the opening of the second proportional valve based on the obtained second flow value, to make the second flow value meet the preset negative pressure flow;

a judgement control unit, used to determine whether the third flow value obtained by the acquisition unit meets the safety flow standard and whether the pressure value meets the safety pressure standard, to control whether the first safety on-off valve and the second safety on-off valve need to be closed; also used to determine whether the inhalation time of the inspiratory tube section has reached the preset positive pressure output time, and whether the exhalation time of the expiratory tube section has reached the preset negative pressure input time, to control the alternate opening and closing of the first high-frequency on-off valve and the second high-frequency on-off valve.

Preferably, the determination of whether the pressure value meets the safety pressure standard includes:

Calculating the average pressure value of the gas supply end obtained before the current moment;

Determining whether the ratio of the absolute difference between the target pressure value of the gas supply end and the average pressure value to the average pressure value is less than a preset threshold; if less than, the pressure value meets the safety pressure standard, if greater than, the pressure value does not meet the safety pressure standard.

Preferably, it also includes:

a blood oxygen collection unit, used to collect the patient's blood oxygen concentration;

a ventilation state judgement unit, based on multiple pressure values detected by the pressure meter during ventilation and multiple blood oxygen concentration values collected by the blood oxygen collection unit, determines a judgement dataset that can represent the ventilation state. Each judgement value in the judgement dataset is used to characterize the probability of abnormal ventilation state at the corresponding time point.

Preferably, determining the judgement dataset that can represent the ventilation state based on multiple pressure values and multiple blood oxygen concentration values includes:

Determining a pressure judgement parameter set and a time judgement parameter set based on multiple pressure values and the target pressure value of the gas supply end;

Determining a blood oxygen concentration judgement parameter set based on multiple blood oxygen concentration values and the patient's target blood oxygen concentration;

Obtaining the judgement dataset that can represent the ventilation state based on the pressure judgement parameter set, time judgement parameter set, and blood oxygen concentration judgement parameter set.

Preferably, the pressure judgement parameter set is determined through the following formula:

$$X_i = \begin{cases} |P_0 - P_i|/P_i, & |P_0 - P_i|/P_i \geq \mu \\ 0, & |P_0 - P_i|/P_i < \mu \end{cases}$$

Where, $X_i$ is the pressure judgement parameter corresponding to the i-th time point, i = 1,2, ... n, $P_0$ is the target pressure value, $P_i$ is the pressure value corresponding to the i-th time point, $\mu$ is the preset threshold in the safety pressure standard;

Then the pressure judgement parameter set A is A = $\{X_1, X_2, ... X_n\}$;

The time judgement parameter set is determined through the following formula:

$$Y_j = T_j/60$$

Where, $Y_j$ is the time judgement parameter when $|P_0 - P_i|/P_i \geq \mu$ occurs for the j-th time, $T_j$ is the duration of the j-th occurrence of $|P_0 - P_i|/P_i \geq \mu$, j = 1,2, ... m;

Then the time judgement parameter set B is B = $\{Y_1, Y_2, ... Y_m\}$;

The blood oxygen concentration judgement parameter set is determined through the following formula:

$$Z_i = |Q_i - Q_M|/|Q_0 - Q_M|$$

Where, $Z_i$ is the blood oxygen concentration judgement parameter corresponding to the i-th time point, $Q_i$ is the blood oxygen concentration value corresponding to the i-th time point, $Q_M$ is the median of multiple blood oxygen concentration values, $Q_0$ is the patient's target blood oxygen concentration;

Then the blood oxygen concentration judgement parameter set C is C = $\{Z_1, Z_2, ... Z_n\}$;

The judgement dataset of the ventilation state is obtained through the following formula:

$$K_i = \begin{cases} \sqrt{(X_i{}^2 + Z_i{}^2 + Y_j{}^2)/3}, & |P_0 - P_i|/P_i \geq \mu \\ \sqrt{(X_i{}^2 + Z_i{}^2)/3}, & |P_0 - P_i|/P_i < \mu \end{cases}$$

Where, $K_i$ is the judgement value of the ventilation state corresponding to the i-th time point;

Then the judgement dataset D of the ventilation state is D = $\{K_1, K_2, ... K_n\}$.

This disclosure also provides a control method for the high-frequency respiratory control system with harmonic-controlled positive and negative pressure ventilation, including:

Step 1: Preset the positive pressure flow and positive pressure output time of the inspiratory tube section, negative pressure flow and negative pressure input time of the expiratory tube section, as well as the safety flow standard and safety pressure standard of the gas supply end;
Step 2: Control the first safety on-off valve and the second safety on-off valve to open;
Step 3: Control the first high-frequency on-off valve to open, and the second high-frequency on-off valve to close;
Step 4: Obtain in real-time the first flow value detected by the first flowmeter and adjust the opening of the first proportional valve to make the first flow value meet the preset positive pressure flow;
Step 5: Obtain in real-time the third flow value detected by the third flowmeter and the pressure value detected by the pressure meter, and determine whether the third flow value and pressure value meet their corresponding preset conditions;
Step 6: If at least one of the third flow value and pressure value does not meet its corresponding preset condition, control the first safety on-off valve and the second safety on-off valve to close; if the third flow value meets the safety flow standard and the pressure value meets the safety pressure standard, continue to determine whether the inhalation time has reached the preset positive pressure output time;
Step 7: If the inhalation time has not yet reached the preset positive pressure output time, return to step 4; if the inhalation time has reached the preset positive pressure output time, control the second high-frequency on-off valve to open and the first high-frequency on-off valve to close;
Step 8: Obtain in real-time the second flow value detected by the second flowmeter and adjust the opening of the second proportional valve to make the second flow value meet the preset negative pressure flow;
Step 9: Obtain in real-time the third flow value detected by the third flowmeter and the pressure value detected by the pressure meter, and determine whether the third flow value and pressure value meet their corresponding preset conditions;
Step 10: If at least one of the third flow value and pressure value does not meet its corresponding preset condition, control the first safety on-off valve and the second safety on-off valve to close; if the third flow value meets the safety flow standard and the pressure value meets the safety pressure standard, continue to determine whether the exhalation time has reached the preset negative pressure input time;

Step 11: If the exhalation time has not yet reached the preset negative pressure input time, return to step 8; if the exhalation time has reached the preset negative pressure input time, determine whether the user wants to end ventilation, if not, return to step 2, if yes, control the first safety on-off valve and the second safety on-off valve to close, ending ventilation.

A ventilator, using the high-frequency respiratory control system with harmonic-controlled positive and negative pressure ventilation described in this invention.

Compared to existing technology, this invention includes at least the following beneficial effects:

The high-frequency respiratory control system and ventilator with harmonic-controlled positive and negative pressure ventilation described in this invention use a high-pressure gas source as positive pressure output and a vacuum source as negative pressure input, controlling the alternate opening and closing of the inspiratory and expiratory tube sections through coordinated positive and negative pressure to achieve high-frequency ventilation for patients;

Unlike traditional high-frequency ventilation, this invention uses a vacuum source as negative pressure input, which can effectively control the expiratory volume during the expiration phase, effectively removing carbon dioxide and preventing carbon dioxide retention;

This invention can simplify the structure of the high-frequency gas flow generator, reduce the size of the ventilator, and simplify the ventilation control logic by controlling the alternate opening and closing of the inspiratory and expiratory tube sections. Since high-frequency oscillatory ventilation is easily affected by gas flow, resulting in poor ventilation stability and low tidal volume control accuracy, this invention can accurately control the tidal volume during ventilation without other interfering factors.

For the high-frequency respiratory control system and ventilator with harmonic-controlled positive and negative pressure ventilation described in this invention, other advantages, objectives, and features of this invention will be partially revealed through the following description, and some will be understood by those skilled in the field through the study and practice of this invention.

**Brief Description of Figures**

The figures are used to provide further understanding of this invention, and form part of the specification. They are used together with the embodiments of this invention to explain the invention, and do not constitute limitations on the invention. In the figures:

Figure 1 is a schematic diagram of the high-frequency respiratory control system with harmonic-controlled positive and negative pressure ventilation described in this invention;

Figure 2 is a three-dimensional structural schematic diagram of the high-frequency respiratory control system with harmonic-controlled positive and negative pressure ventilation described in this invention;

Figure 3 is a schematic diagram of the control module in the high-frequency respiratory control system with harmonic-controlled positive and negative pressure ventilation described in this invention;

Figure 4 is a flow chart of the high-frequency respiratory control method with harmonic-controlled positive and negative pressure ventilation described herein.

**Detailed Description of Embodiments**

The following detailed description of the invention is provided in conjunction with the figures and embodiments to enable those skilled in the field to implement it based on the written specification.

It should be understood that the terms "having", "comprising", and "including" used in this document do not exclude the presence or addition of one or more other elements or their combinations.

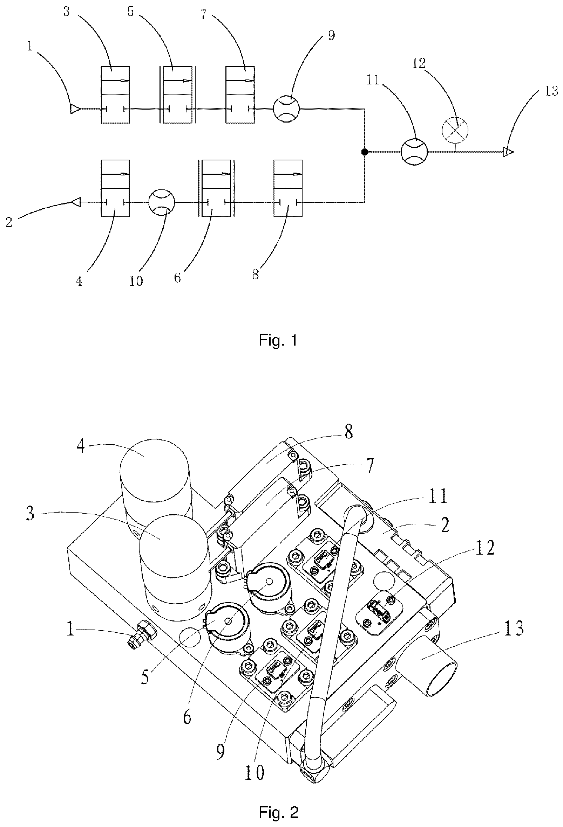

As shown in Figures 1-2, this invention provides a high-frequency respiratory control system with harmonic-controlled positive and negative pressure ventilation, including: an inspiratory tube section and an expiratory tube section, where the inlet A of the inspiratory tube section is connected to a gas source 1, the outlet B of the expiratory tube section is connected to a vacuum source 2, and the outlet C of the inspiratory tube section and the inlet D of the expiratory tube section are both connected to the gas supply end 13 through a third flowmeter 11 and a pressure meter 12; a control module, used to control the alternate opening and closing of the inspiratory and expiratory tube sections, providing high-frequency gas supply to the patient.

This invention uses a high-pressure gas source 1 as positive pressure output and a vacuum source 2 as negative pressure input, controlling the alternate opening and closing of the inspiratory and expiratory tube sections through coordinated positive and negative pressure to achieve high-frequency ventilation for patients;

Unlike traditional high-frequency ventilation, this invention uses a vacuum source 2 as negative pressure input, which can effectively control the expiratory volume during the expiration phase, effectively removing carbon dioxide and preventing carbon dioxide retention;

The vacuum source 2 is one of either a connection to a vacuum pipeline or a vacuum generating device; where the vacuum generating device can be a vacuum pump, vacuum generator, etc.;

This invention can simplify the structure of the high-frequency gas flow generator, reduce the size of the ventilator, and simplify the ventilation control logic by controlling the alternate opening and closing of the inspiratory and expiratory tube sections. Since high-frequency oscillatory ventilation is easily affected by gas flow, resulting in poor ventilation stability and low tidal volume control accuracy, this invention can accurately control the tidal volume during ventilation without other interfering factors.

Furthermore, the inspiratory tube section includes: a first safety on-off valve 3, a first proportional valve 5, a first high-frequency on-off valve 7, and a first flowmeter 9 connected in sequence, where the first safety on-off valve 3 is connected to the gas source 1.

The first safety on-off valve 3 controls whether the gas source 1 is connected to the inspiratory tube section, the first proportional valve 5 and the first flowmeter 9 work together to adjust and detect the gas flow through the inspiratory tube section, and the first high-frequency on-off valve 7 is used to switch the connection between the inspiratory tube section and the gas supply end 13 at high frequency during ventilation.

Furthermore, the expiratory tube section includes: a second safety on-off valve **4, a** second flowmeter 10, a second proportional valve 6, and a second high-frequency on-off valve 8 connected in sequence, where the second safety on-off valve 4 is connected to the vacuum source 2.

The second safety on-off valve 4 controls whether the vacuum source 2 is connected to the expiratory tube section, the second proportional valve 6 and the second flowmeter 10 work together to adjust and detect the gas flow through the expiratory tube section, and the second high-frequency on-off valve 8 is used to switch the connection between the expiratory tube section and the gas supply end 13 at high frequency during ventilation.

Furthermore, the gas source 1 is one of a pure oxygen source, a mixed gas source of air and oxygen, or a mixed gas source of carbon dioxide and oxygen.

The gas source 1 is always a high-pressure gas source. One of a pure oxygen source, a mixed gas source of air and oxygen, or a mixed gas source of carbon dioxide and oxygen is selected based on the patient's needs to meet their oxygen requirements.

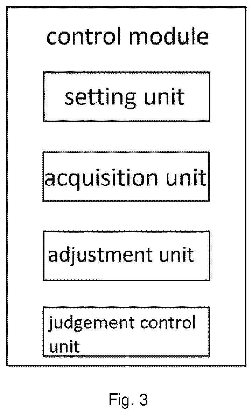

As shown in Figure 3, in one embodiment, the control module includes:

a setting unit, used to preset the required positive pressure flow and positive pressure output time of the inspiratory tube section, negative pressure flow and negative pressure input time of the expiratory tube section, as well as the safety flow standard and safety pressure standard of the gas supply end 13;

an acquisition unit, used to obtain in real-time the first flow value from the first flowmeter 9, the second flow value from the second flowmeter 10, the third flow value from the third flowmeter 11, and the pressure value from the pressure meter 12;

an adjustment unit, used to adjust the opening of the first proportional valve 5 based on the first flow value obtained by the acquisition unit, to make the first flow value meet the preset positive pressure flow; adjust the opening of the second proportional valve 6 based on the obtained second flow value, to make the second flow value meet the preset negative pressure flow;

a judgement control unit, used to determine whether the third flow value obtained by the acquisition unit meets the safety flow standard and whether the pressure value meets the safety pressure standard, to control whether the first safety on-off valve 3 and the second safety on-off valve 4 need to be closed; also used to determine whether the inhalation time of the inspiratory tube section has reached the preset positive pressure output time, and whether the exhalation time of the expiratory tube section has reached the preset negative pressure input time, to control the alternate opening and closing of the first high-frequency on-off valve 7 and the second high-frequency on-off valve 8.

Based on the patient's condition, determine the ratio of inhalation time to exhalation time, ventilation frequency, and tidal volume (tidal volume refers to the volume of gas inhaled or exhaled in each breath), then set the required positive pressure flow and positive pressure output time of the inspiratory tube section, negative pressure flow and negative pressure input time of the expiratory tube section, as well as the safety flow standard and safety pressure standard of the gas supply end 13;

During ventilation, it is necessary to time each inhalation phase (positive pressure) and exhalation phase (negative pressure) to ensure they meet the preset positive pressure output time (inhalation time) and negative pressure input time (exhalation time), and detect the ventilation flow through the first flowmeter 9 and second flowmeter 10 during the inhalation and exhalation phases respectively, adjust the opening of the first proportional valve 5 according to the preset positive pressure flow to make the first flow value meet the preset positive pressure flow, adjust the opening of the second proportional valve 6 according to the preset negative pressure flow to make the second flow value meet the preset negative pressure flow, this can ensure the tidal volume of the patient during inhalation and exhalation, improve the control accuracy of tidal volume, and only need to adjust the proportional valves to stably control the tidal volume;

In addition, a third flowmeter 11 and pressure meter 12 are installed at the gas supply end 13, and safety flow standard and safety pressure standard for the gas supply end 13 are preset, so that the condition of the gas supply end 13 for patient ventilation can be monitored in real-time. During high-frequency ventilation, if the patient's breathing gradually returns to normal, high-frequency ventilation will form resistance to the patient's spontaneous breathing, causing the third flow value and pressure value detected at the gas supply end 13 to exceed the safety flow standard and safety pressure standard. At this time, it is necessary to control the first safety on-off valve 3 and the second safety on-off valve 4 to close, stop ventilation, to prevent harm to the patient.

It should be noted that the safety flow standard can be set as a safe flow range to ensure normal ventilation, and the safety pressure standard can be set as a safe pressure range to ensure normal ventilation, to determine whether the third flow value is within the safe flow range and whether the pressure value is within the safe pressure range.

In one embodiment, the safety pressure standard can also be set as follows, where determining whether the pressure value meets the safety pressure standard includes:

Calculating the average pressure value of the gas supply end 13 obtained before the current moment;

Determining whether the ratio of the absolute difference between the target pressure value of the gas supply end 13 and the average pressure value to the average pressure value is less than a preset threshold; if less than, the pressure value meets the safety pressure standard, if greater than, the pressure value does not meet the safety pressure standard.

It should be noted that the pressure value of the gas supply end 13 obtained before the current moment refers to the average of all pressure values of the gas supply end 13 obtained from the initial moment (initial moment is 0) to the current moment during one inhalation phase or exhalation phase.

Expressed in formula:

$$\left| P_0 - \frac{\sum_0^g P_t}{g} \right| / P_t < \mu$$

Where, during the inhalation phase, t is less than or equal to positive pressure output time, and during the exhalation phase, t is less than or equal to negative pressure input time;

Where, $P_0$ is the target pressure value, $P_t$ is the pressure value of the gas supply end 13 obtained at time t, $g$ is the number of pressure values of the gas supply end 13 obtained at the current moment and before the current moment, $\frac{\sum_0^g P_t}{g}$ is the average pressure value, $\mu$ is the preset threshold;

The pressure value detected in real-time by the pressure meter 12 needs to satisfy the above formula to meet the safety pressure standard. If it does not satisfy the above formula, it is necessary to control the first safety on-off valve 3 and the second safety on-off valve 4 to close, stop ventilation, to prevent harm to the patient.

In one embodiment, it also includes:

a blood oxygen collection unit, used to collect the patient's blood oxygen concentration;

a ventilation state judgement unit, based on multiple pressure values detected by the pressure meter 12 during ventilation and multiple blood oxygen concentration values collected by the blood oxygen collection unit, determines a judgement dataset that can represent the ventilation state. Each judgement value in the judgement dataset is used to characterize the probability of abnormal ventilation state at the corresponding time point.

Determining the judgement dataset that can represent the ventilation state based on multiple pressure values and multiple blood oxygen concentration values includes:

Determining a pressure judgement parameter set and a time judgement parameter set based on multiple pressure values and the target pressure value of the gas supply end 13;

Determining a blood oxygen concentration judgement parameter set based on multiple blood oxygen concentration values and the patient's target blood oxygen concentration;

Obtaining the judgement dataset that can represent the ventilation state based on the pressure judgement parameter set, time judgement parameter set, and blood oxygen concentration judgement parameter set.

Furthermore, the pressure judgement parameter set is determined through the following formula:

$$\mathrm{X}_i = \begin{cases} |P_0 - P_i|/P_i, & |P_0 - P_i|/P_i \geq \mu \\ 0, & |P_0 - P_i|/P_i < \mu \end{cases}$$

Where, $X_i$ is the pressure judgement parameter corresponding to the i-th time point, i = 1,2, ... n, $P_0$ is the target pressure value, $P_i$ is the pressure value corresponding to the i-th time point, $\mu$ is the preset threshold in the safety pressure standard;

Then the pressure judgement parameter set A is A = {$X_1$, $X_2$, ... $X_n$};

The time judgement parameter set is determined through the following formula:

$$\mathrm{Y}_j = \mathrm{T}_j/60$$

Where, $Y_j$ is the time judgement parameter when $|P_0 - P_i|/P_i \geq \mu$ occurs for the j-th time, $T_j$ is the duration of the j-th occurrence of $|P_0 - P_i|/P_i \geq \mu$, j = 1,2, ... m;

Then the time judgement parameter set B is B = {$Y_1$, $Y_2$, ... $Y_m$};

The blood oxygen concentration judgement parameter set is determined through the following formula:

$$\mathrm{Z}_i = |\mathrm{Q}_i - \mathrm{Q}_M|/|\mathrm{Q}_0 - \mathrm{Q}_M|$$

Where, $Z_i$ is the blood oxygen concentration judgement parameter corresponding to the i-th time point, $Q_i$ is the blood oxygen concentration value corresponding to the i-th time point, $Q_M$ is the median of multiple blood oxygen concentration values, $Q_0$ is the patient's target blood oxygen concentration;

Then the blood oxygen concentration judgement parameter set C is C = {$Z_1$, $Z_2$, ... $Z_n$};

The judgement dataset of the ventilation state is obtained through the following formula:

$$K_i = \begin{cases} \sqrt{(X_i{}^2 + Z_i{}^2 + Y_j{}^2)/3}, & |P_0 - P_i|/P_i \geq \mu \\ \sqrt{(X_i{}^2 + Z_i{}^2)/3}, & |P_0 - P_i|/P_i < \mu \end{cases}$$

Where, $K_i$ is the judgement value of the ventilation state corresponding to the i-th time point;

Then the judgement dataset D of the ventilation state is D = {$K_1$, $K_2$, ... $K_n$}.

Where the pressure judgement parameter and time judgement parameter can represent the degree of pressure abnormality at the gas supply end 13, and the blood oxygen concentration judgement parameter can represent the degree of abnormality in the patient's blood oxygen concentration.

The number of judgement values in the judgement dataset increases with ventilation time, where a larger numerical value of the judgement value indicates a higher probability of abnormal ventilation state;

An abnormal threshold can be set, if the judgement value corresponding to a certain time point is higher than the abnormal threshold, then this time point's judgement value is marked as an abnormal judgement value;

If the number of abnormal judgement values is small and discontinuous in time, it indicates that the ventilation state is normal; if the number of abnormal judgement values is large and discontinuous in time, attention should be paid or timely checks should be made on the working condition of devices used for monitoring or implementing the ventilation process, such as various flowmeters, pressure meters, and blood oxygen collection units, to provide early warning. This is because when the first flowmeter 9 or the second flowmeter 10 works abnormally, the low detection accuracy will affect the adjustment of the proportional valves, resulting in low actual ventilation flow accuracy, that is, not meeting the preset positive pressure flow or negative pressure flow, which in turn leads to pressure abnormalities at the gas supply end 13. This will also affect the patient's blood oxygen concentration. Therefore, the judgement dataset can represent the ventilation state of the entire ventilation process, improving the accuracy of tidal volume supply during ventilation and ensuring the safety of ventilation.

The method for determining whether the number of abnormal judgement values is large or small is to calculate the ratio of abnormal judgement values to all judgement values in the judgement dataset, and set a preset ratio (e.g., 1/8). If the ratio is less than the preset ratio, the number of abnormal judgement values is considered small; if the ratio is greater than or equal to the preset ratio, the number of abnormal judgement values is considered large.

If a judgement value at a certain time point is an abnormal judgement value, and the detection values of the third flowmeter 11 and pressure meter 12 have not yet exceeded the safety flow standard and safety pressure standard that would cause the first safety on-off valve 3 and the second safety on-off valve 4 to close, then continue to monitor the judgement values after this time point. If all judgement values after this time point within a monitoring time period are abnormal judgement values, then examine the magnitude of the pressure judgement parameters and blood oxygen concentration judgement parameters in the abnormal judgement values. Based on whichever of the two has a higher degree of abnormality, make adaptive adjustments to the ventilation process. Adjustments to the ventilation process include, but are not limited to, adjusting the positive pressure flow of the inspiratory tube section (opening of the first proportional valve 5, setting the positive pressure output time, adjusting the negative pressure flow of the expiratory tube section (opening of the second proportional valve 6, setting the negative pressure input time, setting the tidal volume, adjusting the oxygen content of the gas source 1 (can be achieved by switching gas sources), and adjusting the ventilation frequency, etc.; This improves the accuracy of tidal volume supply and enhances the stability of the patient's blood oxygen concentration.

By monitoring the ventilation state throughout the entire process, a judgement dataset that can represent the ventilation state can be obtained, which can provide a basis for adjusting the parameters of the ventilation process. It can also provide timely warnings about the working condition of devices used for monitoring and implementing the ventilation process, allowing for timely inspection or replacement to ensure the accuracy of detection by various flowmeters and pressure meters, improve the accuracy of tidal volume supply during the ventilation process, and ensure the safety of ventilation.

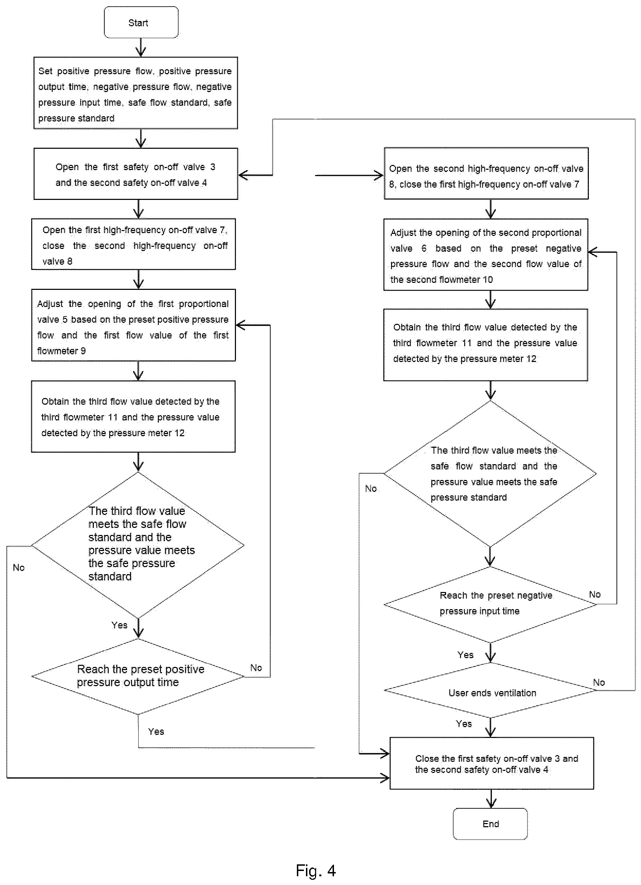

As shown in Figure 4, this disclosure also provides a control method for the high-frequency respiratory control system with harmonic-controlled positive and negative pressure ventilation, including:

Step 1: Preset the positive pressure flow and positive pressure output time of the inspiratory tube section, negative pressure flow and negative pressure input time of the expiratory tube section, as well as the safety flow standard and safety pressure standard of the gas supply end 13;

Step 2: Control the first safety on-off valve 3 and the second safety on-off valve 4 to open;

Step 3: Control the first high-frequency on-off valve 7 to open, and the second high-frequency on-off valve 8 to close;

Step 4: Obtain in real-time the first flow value detected by the first flowmeter 9 and adjust the opening of the first proportional valve 5 to make the first flow value meet the preset positive pressure flow;

Step 5: Obtain in real-time the third flow value detected by the third flowmeter 11 and the pressure value detected by the pressure meter 12, and determine whether the third flow value and pressure value meet their corresponding preset conditions;

Step 6: If at least one of the third flow value and pressure value does not meet its corresponding preset condition, control the first safety on-off valve 3 and the second safety on-off valve 4 to close; if the third flow value meets the safety flow standard and the pressure value meets the safety pressure standard, continue to determine whether the inhalation time has reached the preset positive pressure output time;

Step 7: If the inhalation time has not yet reached the preset positive pressure output time, return to step 4; if the inhalation time has reached the preset positive pressure output time, control the second high-frequency on-off valve 8 to open and the first high-frequency on-off valve 7 to close;

Step 8: Obtain in real-time the second flow value detected by the second flowmeter 10 and adjust the opening of the second proportional valve 6 to make the second flow value meet the preset negative pressure flow;

Step 9: Obtain in real-time the third flow value detected by the third flowmeter 11 and the pressure value detected by the pressure meter 12, and determine whether the third flow value and pressure value meet their corresponding preset conditions;

Step 10: If at least one of the third flow value and pressure value does not meet its corresponding preset condition, control the first safety on-off valve 3 and the second safety on-off valve 4 to close; if the third flow value meets the safety flow standard and the pressure value meets the safety pressure standard, continue to determine whether the exhalation time has reached the preset negative pressure input time;

Step 11: If the exhalation time has not yet reached the preset negative pressure input time, return to step 8; if the exhalation time has reached the preset negative pressure input time, determine whether the user wants to end ventilation, if not, return to step 2, if yes, control the first safety on-off valve 3 and the second safety on-off valve 4 to close, ending ventilation.

Through the above control method, coordinated control of positive and negative pressure can be used to achieve high-frequency ventilation. The combination of flowmeters and proportional valves can achieve precise and stable control of tidal volume, improving control accuracy. Using a vacuum source 2 as negative pressure input can effectively remove carbon dioxide during the exhalation phase, preventing carbon dioxide retention from being re-inhaled by the patient. The set safety flow standard and safety pressure standard can promptly control the first safety on-off valve 3 and the second safety on-off valve 4 to close, preventing inappropriate ventilation from causing harm to the patient.

This invention also provides a ventilator, using the high-frequency respiratory control system with harmonic-controlled positive and negative pressure ventilation described in this invention, including: an inspiratory tube section and an expiratory tube section, where the inlet A of the inspiratory tube section is connected to a gas source 1, the outlet B of the expiratory tube section is connected to a vacuum source 2, and the outlet C of the inspiratory tube section and the inlet D of the expiratory tube section are both connected to the gas supply end 13 through a third flowmeter 11 and a pressure meter 12; the inspiratory tube section includes: a first safety on-off valve 3, a first proportional valve 5, a first high-frequency on-off valve 7, and a first flowmeter 9 connected in sequence, where the first safety on-off valve 3 is connected to the gas source 1; the expiratory tube section includes: a second safety on-off valve 4, a second flowmeter 10, a second proportional valve 6, and a second high-frequency on-off valve 8 connected in sequence, where the second safety on-off valve 4 is connected to the vacuum source 2.

The ventilator described in this invention achieves high-frequency ventilation through coordinated control of positive and negative pressure, using a vacuum source 2 as negative pressure input, which can effectively remove carbon dioxide and prevent retention. The ventilator with this structure can simplify the design, reduce size, and be more convenient for use and movement.

In the description of this invention, it should be understood that terms such as "center", "longitudinal", "transverse", "length", "width", "thickness", "upper", "lower", "front", "back", "left", "right", "vertical", "horizontal", "top", "bottom", "inner", "outer", "clockwise", "counterclockwise", "axial", "radial", "circumferential", etc., indicating orientation or positional relationships are based on the orientation or positional relationships shown in the figures. They are only for the convenience of description and simplification of the invention, and do not indicate or imply that the referred device or element must have a specific orientation, be constructed or operated in a specific orientation. Therefore, they should not be understood as limitations on the invention.

In this invention, unless otherwise explicitly specified and limited, terms such as "installed", "connected", "fixed", etc., should be understood in a broad sense. For example, it can be a fixed connection or a detachable connection, or an integral formation; it can be a mechanical connection or an electrical connection or able to communicate with each other; it can be a direct connection or an indirect connection through an intermediate medium, or an internal communication between two elements or an interaction relationship between two elements. For those skilled in the field, the specific meaning of the above terms in this invention can be understood according to specific situations.

Although the embodiments of this invention have been disclosed as above, they are not limited to the applications listed in the specification and embodiments. The invention is defined by the appended claims.

**Claims**

1.  A high-frequency respiratory control system with harmonic-controlled positive and negative pressure ventilation, comprising: an inspiratory tube section and an expiratory tube section, where the inlet A of the inspiratory tube section is connected to a gas source (1), the outlet B of the expiratory tube section is connected to a vacuum source (2), and the outlet C of the inspiratory tube section and the inlet D of the expiratory tube section are both connected to the gas supply end (13) through a third flowmeter (11) and a pressure meter (12); a control module, used to control the alternate opening and closing of the inspiratory and expiratory tube sections, providing high-frequency gas supply to the patient;

    the inspiratory tube section includes: a first safety on-off valve (3), a first proportional valve (5), a first high-frequency on-off valve (7), and a first flowmeter (9) connected in sequence, where the first safety on-off valve (3) is connected to the gas source (1);
    the expiratory tube section includes: a second safety on-off valve (4), a second flowmeter (10), a second proportional valve (6), and a second high-frequency on-off valve (8) connected in sequence, where the second safety on-off valve (4) is connected to the vacuum source (2); the control module includes:

        a setting unit, used to preset the required positive pressure flow and positive pressure output time of the inspiratory tube section, negative pressure flow and negative pressure input time of the expiratory tube section, as well as the safety flow standard and safety pressure standard of the gas supply end (13);
        an acquisition unit, used to obtain in real-time the first flow value from the first flowmeter (9), the second flow value from the second flowmeter (10), the third flow value from the third flowmeter (11), and the pressure value from the pressure meter (12);
        an adjustment unit, used to adjust the opening of the first proportional valve (5) based on the first flow value obtained by the acquisition unit, to make the first flow value meet the preset positive pressure flow; adjust the opening of the second proportional valve (6) based on the obtained second flow value, to make the second flow value meet the preset negative pressure flow;
        a judgement control unit, used to determine whether the third flow value obtained by the acquisition unit meets the safety flow standard and whether the pressure value meets the safety pressure standard, to control whether the first safety on-off valve (3) and the second safety on-off valve (4) need to be closed; also used to determine whether the inhalation time of the inspiratory tube section has reached the preset positive pressure output time, and whether the exhalation time of the expiratory tube section has reached the preset negative pressure input time, to control the alternate opening and closing of the first high-frequency on-off valve (7) and the second high-frequency on-off valve (8).

2.  The high-frequency respiratory control system with harmonic-controlled positive and negative pressure ventilation according to claim 1, wherein the gas source (1) is one of a pure oxygen source, a mixed gas source of air and oxygen, or a mixed gas source of carbon dioxide and oxygen.

3.  The high-frequency respiratory control system with harmonic-controlled positive and negative pressure ventilation according to claim 1, wherein the determination of whether the pressure value meets the safety pressure standard includes:

        calculating the average pressure value of the gas supply end (13) obtained before the current moment;
        determining whether the ratio of the absolute difference between a target pressure value of the gas supply end (13) and the average pressure value to the average pressure value is less than a preset threshold; if less than, the pressure value meets the safety pressure standard, if greater than, the pressure value does not meet the safety pressure standard.

4. The high-frequency respiratory control system with harmonic-controlled positive and negative pressure ventilation according to claim 1, further comprising:

a blood oxygen collection unit, used to collect the patient's blood oxygen concentration;
a ventilation state judgement unit, based on multiple pressure values detected by the pressure meter (12) during ventilation and multiple blood oxygen concentration values collected by the blood oxygen collection unit, determines a judgement dataset that can represent the ventilation state. Each judgement value in the judgement dataset is used to characterize the probability of abnormal ventilation state at the corresponding time point.

5. The high-frequency respiratory control system with harmonic-controlled positive and negative pressure ventilation according to claim 4, wherein determining the judgement dataset that can represent the ventilation state based on multiple pressure values and multiple blood oxygen concentration values includes:

determining a pressure judgement parameter set and a time judgement parameter set based on multiple pressure values and a target pressure value of the gas supply end (13);
determining a blood oxygen concentration judgement parameter set based on multiple blood oxygen concentration values and the patient's target blood oxygen concentration;
obtaining the judgement dataset that can represent the ventilation state based on the pressure judgement parameter set, time judgement parameter set, and blood oxygen concentration judgement parameter set.

6. A ventilator using the high-frequency respiratory control system with harmonic-controlled positive and negative pressure ventilation according to any one of claims 1-5.

**Patentansprüche**

1. Hochfrequenz-Atemkontrollsystem mit harmonisch gesteuerter Über- und Unterdruckbeatmung, umfassend: einen Inspirationsschlauchabschnitt und einen Exspirationsschlauchabschnitt, wobei der Einlass A des Inspirationsschlauchabschnitts mit einer Gasquelle (1) verbunden ist, der Auslass B des Exspirationsschlauchabschnitts mit einer Vakuumquelle (2) verbunden ist und der Auslass C des Inspirationsschlauchabschnitts und der Einlass D des Exspirationsschlauchabschnitts beide über einen dritten Durchflussmesser (11) und einen Druckmesser (12) mit dem Gasversorgungsende (13) verbunden sind; ein Steuermodul, das das abwechselnde Öffnen und Schließen des Inspirations- und des Exspirationsschlauchabschnitts steuert und dem Patienten eine Hochfrequenz-Gasversorgung bereitstellt;
wobei der Inspirationsschlauchabschnitt Folgendes enthält:

ein erstes Sicherheits-Ein-/Aus-Ventil (3), ein erstes Proportionalventil (5), ein erstes Hochfrequenz-Ein-/Aus-Ventil (7) und einen ersten Durchflussmesser (9), die in Reihe geschaltet sind, wobei das erste Sicherheits-Ein-/Aus-Ventil (3) mit der Gasquelle (1) verbunden ist;
wobei der Exspirationsschlauchabschnitt Folgendes enthält:

ein zweites Sicherheits-Ein-/Aus-Ventil (4), einen zweiten Durchflussmesser (10), ein zweites Proportionalventil (6) und ein zweites Hochfrequenz-Ein-/Aus-Ventil (8), die in Reihe geschaltet sind, wobei das zweite Sicherheits-Ein-/Aus-Ventil (4) mit der Vakuumquelle (2) verbunden ist;
wobei das Steuermodul Folgendes enthält:

eine Einstelleinheit, die verwendet wird, um den erforderlichen positiven Druckfluss und die positive Druckausgabezeit des Inspirationsschlauchabschnitts, den negativen Druckfluss und die negative Druckeingabezeit des Exspirationsschlauchabschnitts sowie den Sicherheitsdurchflussstandard und de Sicherheitsdruckstandard des Gasversorgungsendes (13) voreinzustellen;
eine Erfassungseinheit, die verwendet wird, um in Echtzeit den ersten Durchflusswert von dem ersten Durchflussmesser (9), den zweiten Durchflusswert von dem zweiten Durchflussmesser (10), den dritten Durchflusswert von dem dritten Durchflussmesser (11) und den Druckwert von dem Druckmesser (12) zu erhalten;
eine Anpassungseinheit, die verwendet wird, um die Öffnung des ersten Proportionalventils (5) basierend auf dem ersten von der Erfassungseinheit erhaltenen Durchflusswert anzupassen, damit der erste Durchflusswert dem voreingestellten positiven Druckfluss entspricht; die Öffnung des zweiten Proportionalventils (6) basierend auf dem erhaltenen zweiten Durchflusswert anzupassen, damit der zweite Durchflusswert dem voreingestellten negativen Druckfluss entspricht;

eine Beurteilungssteuereinheit, die verwendet wird, um zu bestimmen, ob der von der Erfassungseinheit erhaltene dritte Durchflusswert dem Sicherheitsdurchflussstandard entspricht und ob der Druckwert dem Sicherheitsdruckstandard entspricht, um zu steuern, ob das erste Sicherheits-Ein-/Aus-Ventil (3) und das zweite Sicherheits-Ein-/Aus-Ventil (4) geschlossen werden müssen;

und auch verwendet wird, um zu bestimmen, ob die Inspirationszeit des Inspirationsschlauchabschnitts die voreingestellte positive Druckausgabezeit erreicht hat und ob die Exspirationszeit des Exspirationsschlauchabschnitts die voreingestellte negative Druckeingabezeit erreicht hat, um das abwechselnde Öffnen und Schließen des ersten Hochfrequenz-Ein-/Aus-Ventils (7) und des zweiten Hochfrequenz-Ein-/Aus-Ventils (8) zu steuern.

2. Hochfrequenz-Atemkontrollsystem mit harmonisch gesteuerter Über- und Unterdruckbeatmung nach Anspruch 1, wobei die Gasquelle (1) eine reine Sauerstoffquelle, eine gemischte Gasquelle von Luft und Sauerstoff oder eine gemischte Gasquelle von Kohlendioxid und Sauerstoff ist.

3. Hochfrequenz-Atemkontrollsystem mit harmonisch gesteuerter Über- und Unterdruckbeatmung nach Anspruch 1, wobei die Bestimmung, ob der Druckwert den Sicherheitsdruckstandard erfüllt, Folgendes enthält:

Berechnen des durchschnittlichen Druckwerts des Gasversorgungsendes (13), der vor dem aktuellen Moment erhalten wurde;

Bestimmen, ob das Verhältnis der absoluten Differenz zwischen einem Zieldruckwert des Gasversorgungsendes (13) und dem mittleren Druckwert zu dem mittleren Druckwert kleiner als ein voreingestellter Schwellenwert ist; wenn kleiner, entspricht der Druckwert dem Sicherheitsdruckstandard, wenn größer, entspricht der Druckwert nicht dem Sicherheitsdruckstandard.

4. Hochfrequenz-Atemkontrollsystem mit harmonisch gesteuerter Über- und Unterdruckbeatmung nach Anspruch 1, ferner umfassend:

eine Blutsauerstoff-Entnahmeeinheit, die verwendet wird, um die Sauerstoffkonzentration in dem Blut des Patienten zu entnehmen;

eine Beatmungszustand-Beurteilungseinheit, die basierend auf mehreren Druckwerten, die von dem Druckmesser (12) während der Beatmung erkannt werden, und mehreren Blutsauerstoffkonzentrationswerten, die von der Blutsauerstoff-Entnahmeeinheit entnommen werden, einen Beurteilungsdatensatz bestimmt, der den Beatmungszustand darstellen kann. Jeder Beurteilungswert in dem Beurteilungsdatensatz wird verwendet, um die Wahrscheinlichkeit eines abnormalen Beatmungszustands zu dem entsprechenden Zeitpunkt zu charakterisieren.

5. Hochfrequenz-Atemkontrollsystem mit harmonisch gesteuerter Über- und Unterdruckbeatmung nach Anspruch 4, wobei das Bestimmen des Beurteilungsdatensatzes, der den Beatmungszustand basierend auf mehreren Druckwerten und mehreren Blutsauerstoffkonzentrationswerten darstellen kann, Folgendes enthält:

Bestimmen eines Parametersatzes für die Druckbewertung und eines Parametersatzes für die Zeitbewertung basierend auf mehreren Druckwerten und einem Zieldruckwert des Gasversorgungsendes (13);

Bestimmen eines Parametersatzes für die Blutsauerstoffkonzentrationsbewertung basierend auf mehreren Blutsauerstoffkonzentrationswerten und der Ziel-Blutsauerstoffkonzentration des Patienten;

Erhalten des Bewertungsdatensatzes, der den Beatmungszustand basierend auf dem Parametersatz für die Druckbewertung, dem Parametersatz für die Zeitbewertung und dem Parametersatz für die Blutsauerstoffkonzentrationsbewertung darstellen kann.

6. Beatmungsgerät unter Verwendung des Hochfrequenz-Atemkontrollsystems mit harmonisch gesteuerter Über- und Unterdruckbeatmung nach einem der Ansprüche 1 bis 5.

**Revendications**

1. Système de commande respiratoire haute fréquence avec ventilation à pression positive et négative commandée par harmoniques, comportant : une section de tuyau inspiratoire et une section de tuyau expiratoire, où l'entrée A de la section de tuyau inspiratoire est raccordée à une source de gaz (1), la sortie B de la section de tuyau expiratoire est raccordée à une source de vide (2) et la sortie C de la section de tuyau inspiratoire et l'entrée D de la section de tuyau expiratoire sont toutes deux raccordées à l'extrémité d'alimentation en gaz (13) par l'intermédiaire d'un troisième débitmètre (11) et d'un manomètre (12) ; un module de commande, utilisé pour commander l'ouverture et la fermeture alternées des sections de tuyaux inspiratoire et expiratoire, fournissant une alimentation en gaz haute fréquence au patient ;

la section de tuyau inspiratoire inclut : une première valve marche/arrêt de sécurité (3), une première valve proportionnelle (5), une première valve marche/arrêt haute fréquence (7) et un premier débitmètre (9) raccordés en séquence, où la première valve marche/arrêt de sécurité (3) est raccordée à la source de gaz (1) ;

la section de tuyau expiratoire inclut : une deuxième valve marche/arrêt de sécurité (4), un deuxième débitmètre (10), une deuxième valve proportionnelle (6) et une deuxième valve marche/arrêt haute fréquence (8) raccordés en séquence, où la deuxième valve marche/arrêt de sécurité (4) est raccordée à la source de vide (2) ;

le module de commande inclut :

une unité de réglage, utilisée pour prérégler un débit de pression positive requis et un temps de sortie de pression positive de la section de tuyau inspiratoire, un débit de pression négative et un temps d'entrée de pression négative de la section de tuyau expiratoire, ainsi que la norme de débit de sécurité et la norme de pression de sécurité de l'extrémité d'alimentation en gaz (13) ;

une unité d'acquisition, utilisée pour obtenir en temps réel la première valeur de débit du premier débitmètre (9), la deuxième valeur de débit du deuxième débitmètre (10), la troisième valeur de débit du troisième débitmètre (11) et la valeur de pression du manomètre (12) ;

une unité d'ajustement, utilisée pour ajuster l'ouverture de la première valve proportionnelle (5) sur la base de la première valeur de débit obtenue par l'unité d'acquisition, afin que la première valeur de débit corresponde au débit de pression positive préréglé ; ajuster l'ouverture de la deuxième valve proportionnelle (6) sur la base de la deuxième valeur de débit obtenue, afin que la deuxième valeur de débit corresponde au débit de pression négative préréglé ;

une unité de commande d'évaluation, utilisée pour déterminer si la troisième valeur de débit obtenue par l'unité d'acquisition satisfait à la norme de débit de sécurité et si la valeur de pression satisfait à la norme de pression de sécurité, pour décider si la première valve marche-arrêt de sécurité (3) et la deuxième valve marche-arrêt de sécurité (4) doivent être fermées ou non ;

également utilisée pour déterminer si le temps d'inspiration de la section de tuyau inspiratoire a atteint le temps de sortie de pression positive préréglé, et si le temps d'expiration de la section de tuyau expiratoire a atteint le temps d'entrée de pression négative préréglé, pour commander l'ouverture et la fermeture alternées de la première valve marche-arrêt haute fréquence (7) et de la deuxième valve marche-arrêt haute fréquence (8).

2. Système de commande respiratoire haute fréquence avec ventilation à pression positive et négative commandée par harmoniques selon la revendication 1, dans lequel la source de gaz (1) est l'une parmi une source d'oxygène pur, une source de gaz mixte d'air et d'oxygène ou une source de gaz mixte de dioxyde de carbone et d'oxygène.

3. Système de commande respiratoire haute fréquence avec ventilation à pression positive et négative commandée par harmoniques selon la revendication 1, dans lequel le fait de déterminer si la valeur de pression satisfait ou non à la norme de pression de sécurité inclut :

le calcul de la valeur de pression moyenne de l'extrémité d'alimentation en gaz (13) obtenue avant le moment actuel ;

le fait de déterminer si le rapport de la différence absolue entre une valeur de pression cible de l'extrémité d'alimentation en gaz (13) et la valeur de pression moyenne à la valeur de pression moyenne est inférieur à un seuil prédéfini ; s'il est inférieur, la valeur de pression satisfait à la norme de pression de sécurité, s'il est supérieur, la valeur de pression ne satisfait pas à la norme de pression de sécurité.

4. Système de commande respiratoire haute fréquence avec ventilation à pression positive et négative commandée par harmoniques selon la revendication 1, comportant en outre :

une unité de collecte d'oxygène dans le sang, utilisée pour collecter la concentration en oxygène dans le sang du patient ;

une unité d'évaluation d'état de ventilation, sur la base de multiples valeurs de pression détectées par le manomètre (12) pendant la ventilation et de multiples valeurs de concentration en oxygène dans le sang collectées par l'unité de collecte d'oxygène dans le sang, détermine un ensemble de données d'évaluation qui peut représenter l'état de ventilation. Chaque valeur d'évaluation dans l'ensemble de données d'évaluation est utilisée pour caractériser la probabilité d'un état de ventilation anormal à l'instant correspondant.

5. Système de commande respiratoire haute fréquence avec ventilation à pression positive et négative commandée par harmoniques selon la revendication 4, dans lequel la détermination de l'ensemble de données d'évaluation qui peut représenter l'état de ventilation sur la base de multiples valeurs de pression et de multiples valeurs de concentration en oxygène dans le sang inclut :

la détermination d'un ensemble de paramètres d'évaluation de pression et d'un ensemble de paramètres d'évaluation de temps sur la base de multiples valeurs de pression et d'une valeur de pression cible de l'extrémité d'alimentation en gaz (13) ;
la détermination d'un ensemble de paramètres d'évaluation de concentration en oxygène dans le sang sur la base de multiples valeurs de concentration en oxygène dans le sang et de la concentration en oxygène dans le sang cible du patient ;
l'obtention de l'ensemble de données d'évaluation qui peut représenter l'état de ventilation sur la base de l'ensemble de paramètres d'évaluation de pression, de l'ensemble de paramètres d'évaluation de temps et de l'ensemble de paramètres d'évaluation de concentration d'oxygène dans le sang.

6. Ventilateur utilisant le système de commande respiratoire haute fréquence avec ventilation à pression positive et négative commandée par harmoniques selon l'une quelconque des revendications 1 à 5.

Fig. 1

Fig. 2

control module

setting unit

acquisition unit

adjustment unit

judgement control unit

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021179215 A1